## Europäisches Patentamt
## European Patent Office
## Office européen des brevets

(11) Veröffentlichungsnummer: **0 281 679 B1**

# EUROPÄISCHE PATENTSCHRIFT

(45) Veröffentlichungstag der Patentschrift: 26.06.91

(51) Int. Cl.⁵: **C07G 17/00, A61K 35/10**

(21) Anmeldenummer: **87118288.7**

(22) Anmeldetag: **10.12.87**

Die Akte enthält technische Angaben, die nach dem Eingang der Anmeldung eingereicht wurden und die nicht in dieser Patentschrift enthalten sind.

(54) **Niedermolekulare Alkalihuminate, Verfahren zur ihrer Herstellung und Verwendung.**

(30) Priorität: **12.03.87 DE 3707909**

(43) Veröffentlichungstag der Anmeldung:
**14.09.88 Patentblatt 88/37**

(45) Bekanntmachung des Hinweises auf die Patenterteilung:
**26.06.91 Patentblatt 91/26**

(84) Benannte Vertragsstaaten:
**AT BE CH DE ES FR GB GR IT LI LU NL SE**

(56) Entgegenhaltungen:
**EP-A- 281 678**
**DE-B- 1 251 319**
**DE-C- 712 697**

**CHEMICAL ABSTRACTS, Band 103, Nr. 13, Nr. 98707D, 30. September 1985, Seite 56, Columbus, Ohio, US; Z. SHUSHI et al.: "Effects of sodium humate from air-slacked coal on wound healing", & YAOXUE TONGBAO 1984, 19(5), 285-8**

(73) Patentinhaber: **RÜTGERSWERKE AKTIENGESELLSCHAFT**
**Mainzer Landstrasse 217**
**W-6000 Frankfurt am Main 1(DE)**

(72) Erfinder: **Seubert, Bernhard**
**Meisenweg 15**
**W-6803 Edingen-Neckarhausen 1(DE)**
Erfinder: **Beilharz, Helmut, Dr.**
**Steinachstrasse 14**
**W-6905 Schriesheim(DE)**
Erfinder: **Fickert, Werner, Dr.**
**Stockacher Strasse 14**
**W-6800 Mannheim 61(DE)**
Erfinder: **Jeromin, Günter, Dr.**
**Bergstrasse 14**
**W-6900 Heidelberg(DE)**
Erfinder: **Spitaler, Ulrich, Dr.**
**Dürkheimer Hohl 2**
**W-6713 Freinsheim(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

PLANT AND OIL, Band 66, 1982, Seiten 205-215, Dr. W. Junk Publishers, Den Haag, NL; N.R. CURVETTO et al.: "Electrophoretic subfractionation of low and high molecular weight humic acids fractions"

FUEL, Band 60, August 1981, Seiten 685-688, E.M. BALABANOVA-RADONOVA et al.: "Low-molecular-weight fractions of Bulgarian lignite humic acids"

Lantbrukshögskolans Annaler, Band 35, 1969, Seiten 815-822; I. Lindquist et al.: "On the formation of humic acids by oxidation of phenolic compounds"

Römpps Chemie-Lexikon, ISBN 3-440-04510-2; Seite 1771

**Beschreibung**

Die Erfindung betrifft ein Verfahren zur Herstellung von als Alkalihuminate bezeichneten Alkali- oder Ammoniumsalzen von Huminsäuren mit niedrigem Molekulargewicht.

Huminsäuren sind Gemische ähnlich aufgebauter, organischer höher- bzw. hochmolekularer Verbindungen, deren Molekulargewichte in der Literatur als im Bereich von 2000 bis zu über 200000 liegend angegeben werden.

Diesen Huminsäuren oder allgemein Huminstoffen, die in Mooren, Torf, Farberden oder in huminstoffhaltigen Wassern vorkommen, werden heilende Wirkungen zugesprochen (vergleiche Ziechmann, Therapiewoche 28 (1978), 1199-1211). Andererseits sind die Huminsäuren bzw. ihre Salze parenteral als toxisch anzusehen (vergleiche Kühnert et al., Arch. exper. Vet. med. 36 (1982), 169-177), weshalb die Huminstoffe wohl, abgesehen von Moorbädern, vorwiegend in der Veterinärmedizin eingesetzt werden.

Es ist daher Aufgabe der Erfindung, Huminstoffe bereitzustellen, die als parenteral wesentlich weniger toxisch anzusehen sind, deren Zubereitung eine gleichmäßige pharmazeutische Wirksamkeit zeigen und langfristig lagerstabil sind, sich demnach in der Humanmedizin bedenkenlos einsetzen lassen.

Die Lösung der Aufgabe erfolgt durch ein Verfahren zur Herstellung von Alkali- oder Ammoniumsalzen niedermolekularer Huminsäuren gemäß der Ansprüche 1 bis 4 und ihre medizinische Verwendung gemäß der Ansprüche 5 bis 7.

Es ist bekannt (Ziechmann, Huminstoffe, Verlag Chemie 1980, 223 ff.), Huminsäuren mit verschiedenen Methoden wie Elektrophorese, Ultrazentrifugation, Säulenchromatographie oder mittels verschiedener organischer Lösemittel analytisch in verschiedene Fraktionen zu trennen. Die einzelnen Fraktionen unterscheiden sich in ihren Molekulargewichten.

Es wurde nun gefunden, daß sich bei besonderer Aufarbeitung eines bestimmten alkalisch-wäßrigen Extraktes von Huminstoffen eine Fraktion von Alkalihuminaten gewinnen läßt, deren parenterale Toxität wesentlich geringer ist als die beschriebenen Huminate, und die eine gute heilende Wirkung besitzt. Die Huminate dieser Fraktion haben eine mittlere relative Molmasse von 1000 g/mol bei einem Streubereich von 300 bis 1500 g/mol.

Überraschenderweise hat diese Fraktion niedermolekularer Alkalihuminate den zusätzlichen Vorteil, daß sie stabil ist. Obwohl Huminsäuren und Huminate normalerweise einem ständigen Humifizierungsprozeß unterliegen, das heißt ständig weiterpolymerisieren, und obwohl gerade die niedermolekularen Huminstoffe in dieser Hinsicht als besonders reaktiv angesehen werden, werden bei der erfindungsgemäßen Alkalihuminatfraktion auch bei langer Lagerung keine Tendenzen zu einer Weiterpolymerisation beobachtet.

Ausgangsprodukte für die erfindungsgemäßen Huminate können alle huminstoffhaltigen Produkte sein, wie z. B. Torf, Moor, Braunkohle, Farberden oder huminstoffhaltige Wasser bzw. deren Humusschlamm.

Diese huminsäurehaltigen, bevorzugt huminsäurereichen Substrate mit Gehalten von mindestens 5, maximal etwa 50 % Huminsäure, werden in Wasser aufgeschlämmt und mit einer Alkalilauge oder Ammoniumhydroxidlösung versetzt, oder sie werden direkt mit einer Alkalilauge oder Ammoniumhydroxidlösung verrührt. Als Alkalilauge kann die Lösung eines beliebigen Alkalihydroxids eingesetzt werden. Aus wirtschaftlichen Gründen sind aber Kalium-oder Natriumhydroxid bevorzugt. Die Menge an Alkalilauge oder Ammoniumhydroxid ist nicht kritisch. Sie sollte aber in der Größenordnung liegen, daß der pH-Wert der entstehenden Suspension im Bereich von 8 - 9 liegt.

Das Herstellen dieser alkalischen Suspension muß insofern vorsichtig erfolgen, als die Mischung zum Schäumen neigt. Es ist daher günstig, wenn das Einmischen des Substrats in die alkalische Lösung bei leicht erhöhter Temperatur, etwa 25 bis 40 °C, erfolgt.

Sobald die Tendenz zum Schäumen abgeklungen ist, wird die Suspension auf eine Temperatur im Bereich von 45 bis 60 °C erwärmt und bei dieser Temperatur mindestens 24 h, bevorzugt 48 bis 72 h lang gerührt. Danach bleibt die Mischung 2 bis 8, bevorzugt 4 bis 6 Wochen bei Raumtemperatur stehen, wobei ungelöste Partikel sedimentieren und die überstehende Lösung abdekantiert oder abfiltriert wird. Der gleiche Effekt läßt sich alternativ durch eine Grobzentrifugation (1000 - 5000 xg) erreichen. Die resultierende Lösung, wird sodann in einer geeigneten Zentrifuge bei etwa 10000 bis 30000 xg weitergereinigt und damit von feinsten Feststoffen und hochmolekularen Huminaten befreit. Das Zentrifugat wird durch Zugabe von Säure oder Einwirkung von saurem Ionenaustauscher neutralisiert (pH 7), danach mit einem geeigneten Puffer (z. B. Phosphat-, Tris-, Zitronensäure-Puffer) auf einen pH-Wert im Bereich von 6,2 bis 7,2 eingestellt. Dabei fallen weitere säurefällbare Huminstofffraktionen aus. Daher wird diese Lösung erneut bei etwa 10000 bis 30000 xg zentrifugiert.

Die so gereinigte und von hochmolekularen Huminstoffen befreite Huminatlösung wird nun erneut mit Hilfe an sich bekannter Methoden, wie z. B. Ultrazentrifuge, präparative HPLC, Säulen- oder Gelchromatographie, fraktioniert. Das bevorzugte Fraktionierungsverfahren ist die Elektrodialyse.

3

EP 0 281 679 B1

Die Elektrodialyse kann entweder als Nieder-
(20 - 400 V) oder als Hochspannungsversion (5 - 250 KV) durchgeführt werden.

Die erfindungsgemäße Fraktion ist niedermolekularer, hat elektrische Ladungen und wird daher in die Elektrodenkammern des Elektrodialysegeräts transportiert. Eine abschließende Reingewinnung der gewünschten Fraktion kann gegebenenfalls durch zusätzliche Ultrafiltration mit Filtern der Trenngrenze 5000 - 1000 D erfolgen.

Die Erfindung wird durch die nachfolgenden Beispiele näher erläutert.

Beispiel 1

In einem Rührwerksbehälter, der gegen Alkalien beständig ist, werden 100 1 entmineralisiertes Wasser (Leitfähigkeit: unter 5 mikro s/cm; pH-Wert 5-7) und 7,7 kg 50%ige Natronlauge (DAB 8) vermischt. Zu dieser Lösung werden innerhalb von 1 h bei einer Temperatur von 35 bis 40 °C 10 kg Torf zugegeben.

Sobald die Tendenz zum Schäumen abgeklungen ist, wird die Mischung vorsichtig auf 50 °C erwärmt und 48 h lang bei dieser Temperatur gehalten und gerührt.

Anschließend bleibt das Gemisch unter Luftabschluß 4 Wochen bei Raumtemperatur stehen und wird danach über ein Filter von 0,5 mm Porenweite filtriert. Die filtrierte Lösung wird über einen Westfalia-Separator unter Verwendung einer Vollmantel-Kammertrommel bei ca. 20000 xg gereinigt.

Das Zentrifugat wird durch Zugabe von o-Phosphorsäure (DAB 7) auf pH 6,5 eingestellt, dabei gleichzeitig gepuffert und danach erneut bei ca. 20000 xg zentrifugiert.

Aus dieser Lösung wird in einer Anlage zur Elektrodialyse, die mit einer Membrane von 0,025 um aus gesintertem Glas ausgestattet ist, durch Anlegen einer Gleichspannung von 200 V die erfindungsgemäße Fraktion (1) in der Anodenkammer als 5 bis 8%ige braune Lösung erhalten.

Das Retenat wird fortlaufend umgepumpt, um Konzentrationspolarisationen zu vermeiden. Aus der elektrophoretischen Wanderungsgeschwindigkeit wird für die erfindungsgemäß abgetrennten Huminate ein mittleres Molekulargewicht von 1000 bei einem Streubereich von 300 bis 1500 ermittelt. Folgende Werte werden während der laufenden Dialyse kontrolliert:
pH-Wert : 6,4 bis 6,8
Mikrodialysetest : negativ

Bei der Stabilitätskontrolle ist nach 60 Tagen Wechselbelastung 65/4 °C im 12/12 h Rhythmus keine Veränderung der Parameter Gehalt, pH-Wert, Redoxspannung und Mikrodialysetest über die Zufallsschwankungen hinaus feststellbar.

Beispiel 2

In einem Rührwerksbehälter, der gegen Alkalien beständig ist, werden 100 1 entmineralisiertes Wasser (Leitfähigkeit: unter 5 mikro s/cm; pH-Wert 5-7) und 8 1 konzentrierte Ammoniaklösung vermischt. Zu dieser Lösung werden innerhalb von 1 h bei einer Temperatur von 35 bis 40 °C 10 kg Heilerde zugegeben. Sobald die Tendenz zum Schäumen abgeklungen ist, wird die Mischung vorsichtig auf 50 °C erwärmt und 48 h lang bei dieser Temperatur gehalten und gerührt.

Anschließend bleibt das Gemisch unter Luftabschluß 4 Wochen bei Raumtemperatur stehen und wird danach über ein Filter von 0,5 mm Porenweite filtriert. Die filtrierte Lösung wird über einen Westfalia-Separator unter Verwendung einer Vollmantel-Kammertrommel bei ca. 20000 xg gereinigt.

Das Zentrifugat wird durch Zugabe eines stark sauren Ionenaustauscherharzes auf pH 7,0 eingestellt und nach Abfiltrieren des Harzes durch Zugabe von Ammonium-Zitronensäure-Puffer auf pH 6,8 eingestellt und gepuffert und danach erneut bei ca. 20000 xg zentrifugiert.

Aus der resultierenden Lösung wird analog Beispiel 1 durch Elektrodialyse die erfindungsgemäße Fraktion (2) isoliert.Die erhaltene Lösung wird durch Filtrieren durch ein Filter mit der Trenngrenze 5000 - 1000 D hochgereinigt.

Bei der Stabilitätskontrolle ist nach 60 Tagen Wechselbelastung 56/4 °C im 12/12 h Rhythmus keine Veränderung der Parameter Gehalt, pH-Wert, Redoxspannung und Mikrodialysetest über die Zufallsschwankungen hinaus feststellbar.

Beispiel 3

Mit den in den Beispielen 1 und 2 erhaltenen Fraktionen (1) und (2) werden die nachfolgend beschriebenen Untersuchungen durchgeführt und die aufgeführten Ergebnisse erhalten.

4

Toxität:

Bei Injektionen der 1%igen Lösung an Versuchstieren (Mäuse) werden folgende Werte der $LD_{50}$ erhalten.

|        | Fraktion (1)    | Fraktion (2)    |
|--------|-----------------|-----------------|
| s.c.   | 1280 (mg/kg)    | 1115 (mg/kg)    |
| i.p.   | 828             | 770             |
| i.V.   | 670             | 718             |

Stabilität:

Nach einer 6 monatigen Lagerung unter Luftabschluß bei 23 °C sind bei den unter Beispiel 1 und 2 genannten Prüfungen keine Veränderungen feststellbar.

Heilwirkung:
Fibroplastentest

Eine nach Thrypsinbehandlung in Suspension gebrachte Kultur von L - Zellen (Mäusefibroblasten) wird mit 50 ppm niedermolekularem Alkalihuminat versetzt.
Die Kultur wird bei 37 °C unter Verwendung eines handelsüblichen Nährmediums 48 h lang bebrütet.
Parallel hierzu wird als Vergleichsversuch eine analoge Kultur ohne Alkalihuminat gleichermaßen bebrütet. Danach wird die Anzahl der lebenden Zellen in beiden Kulturen bestimmt. In der mit niedermolekularen Alkalihuminaten versetzten Kultur liegt die Zahl der lebenden Zellen um 30 % höher als in der Vergleichskultur.

Wundheilung:

An 2 x 10 haarlosen Mäusen werden mit einem Mikrodermatom oberflächliche Wunden, die nur die obersten Epithelschichten erfaßten, in einer Größe von etwa 50 mm² beigebracht. Bei zehn dieser Mäuse wird die Wunde mit einer 1 %igen Lösung einmal benetzt. Die anderen Mäuse bleiben unbehandelt. Während des Beobachtungszeitraumes von 7 d läßt sich folgendes beobachten: Gegenüber den unbehandelten Mäusen nimmt bei den behandelten Versuchstieren
die Wundflächen rascher ab,
die Wunde trocknet früher ab,
die Granulation setzt früher ein und
die Wunde reinigt sich früher.
Insgesamt ist die Abheilung 2 - 3 d früher als bei den Kontrolltieren zu beobachten.

**Ansprüche**

1.  Verfahren zum Herstellen von Alkali- oder Ammoniumsalzen von Huminsäuren mit einem mittleren Molekulargewicht von 1000 bei einem Streubereich von 300 bis 1500, **dadurch gekennzeichnet**, daß huminstoffhaltige Produkte in alkalischer oder ammoniakalischer wäßriger Lösung aufgeschlämmt werden, die entstehende Suspension von groben Feststoffen abgetrennt und mittels Zentrifugieren von feinsten Feststoffen und hochmolekularen Huminaten befreit, die so erhaltene Lösung neutralisiert und bei einem pH-Wert im Bereich von 6,2 bis 7,2 gepuffert und nochmals durch Zentrifugieren gereinigt und von dieser Lösung eine niedermolekulare Fraktion abgetrennt wird.

2.  Verfahren nach Anspruch 1, **dadurch gekennzeichnet**, daß das Zentrifugierenvor und nach der Säurefällung bei etwa 10000 bis 30000 g erfolgt.

3.  Verfahren nach den Ansprüchen 1 und 2, **dadurch gekennzeichnet**, daß die Abtrennung der niedermolekularen Fraktion mittels nieder- und /oder Elektrodialyse erfolgt.

4. Verfahren nach den Ansprüchen 1 - 3, **dadurch gekennzeichnet,** daß eine abschließende Hochreinigung der niedermolekularen Alkalihuminate durch Filtration durch ein Filter mit der Trenngrenze 5000 - 1000 D. erfolgt.

5. Niedermolekulare Alkali- oder Ammoniumsalze von Huminsäuren, hergestellt nach Anspruch 1, zur Verwendung als Wundheilmittel.

6. Verwendung der nach Anspruch 1 hergestellten niedermolekularen Alkali- oder Ammoniumsalze von Huminsäuren zur Herstellung von Heilmitteln in der Wundheilung.

7. Verwendung der nach Anspruch 1 hergestellten niedermolekularen Alkali- oder Ammoniumsalze zur Herstellung von synthetischen Moorbädern.

## Claims

1. A process for preparing alkali or ammonium salts of humic acids with an average molecular weight of 1000 with a scatter of from 300 to 1500, characterized in that products containing humic matter are suspended in an alkaline or ammoniacal aqueous solution, the suspension formed is separated from coarse solids and is freed of very fine solids and high-molecular huminates by centrifuging, the solution thus obtained neutralized and buffered at a pH value in the range of from 6·2 to 7·2 and once again purified by centrifuging, and a low-molecular fraction separated from this solution.

2. A process according to claim 1, characterized in that the centrifuging takes place before and after the acid precipitation at approximately from 10,000 to 30,000 g.

3. A process according to Claims 1 and 2, characterized in that the separation of the low-molecular fraction is performed by means of low and/or electro-dialysis.

4. A process according to Claims 1 to 3, characterized in that a final high purification of the low-molecular alkali huminates is performed by filtration through a filter with a separation threshold of from 5,000 to 1,000 D.

5. Low-molecular alkali or ammonium salts of humic acids, prepared according to Claim 1, for use as wound healing agents.

6. Use of the low-molecular alkali or ammonium salts of humic acids prepared according to Claim 1 for the preparation of healing agents in wound healing.

7. Use of the low-molecular alkali or ammonium salts of humic acids prepared according to Claim 1 for the preparation of synthetic mud baths.

## Revendications

1. Procédé de préparation de sels alcalins ou d'ammonium d'acides humiques ayant une masse moléculaire moyenne de 1000 pour un domaine de dispersion de 300 à 1500, caractérisé en ce que des produits contenant des matières humiques sont mis en suspension dans une solution aqueuse alcaline ou ammoniacale, que la suspension obtenue est séparée des substances solides grossières et débarrassée par centrifugation des substances solides les plus fines et des humates de masse moléculaire élevée, qu'on neutralise la solution ainsi obtenue et la tamponne à un pH dans le domaine de 6,2 à 7,2, qu'on la purifie encore une fois par centrifugation et qu'on sépare de cette solution une fraction de faible masse moléculaire.

2. Procédé selon la revendication 1, caractérisé en ce que la centrifugation est effectuée avant et après la précipitation par les acides sous environ 10000 à 30000 g.

3. Procédé selon les revendications 1 et 2, caractérisé en ce que la séparation de la fraction de faible masse moléculaire est effectuée par électrodialyse sous basse et/ou haute tension.

4. Procédé suivant les revendications 1 à 3, caractérisé en ce qu'on effectue une purification poussée finale des humates alcalins de faible masse moléculaire par filtration à travers un filtre ayant une limite de séparation de 5000 à 1000 D.

5. Sels alcalins ou d'ammonium d'acides humiques de faible masse moléculaire préparés selon la revendication 1 en vue de l'emploi comme agents cicatrisants.

6. Utilisation des sels alcalins ou d'ammonium d'acides humiques de faible masse moléculaire préparés selon la revendication 1 pour la préparation de médicaments pour la cicatrisation des plaies.

7. Utilisation des sels alcalins ou d'ammonium de faible masse moléculaire préparés selon la revendication 1 pour la préparation de bains de boue synthétiques de haute activité.

7